# FASCICULE DE BREVET EUROPEEN

(11) **EP 1 928 993 B1**
(45) Date de publication et mention de la délivrance du brevet: **02.06.2010**
(21) Numéro de dépôt: 06794312.6
(22) Date de dépôt: 09.08.2006
(51) Int. Cl.: C12H 1/14, A23L 1/275, A61K 31/353, A61K 8/97, A61P 9/00

(54) **COMPOSITION DE TANINS PROANTHOCYANIDIQUES, SON PROCÉDÉ DE FABRICATION ET SES APPLICATIONS.**
PROANTHOCYANIDIN-TANNIN-ZUSAMMENSETZUNG, HERSTELLUNGSVERFAHREN DAFÜR UND VERWENDUNG DAVON
PROANTHOCYANIDIN TANNIN COMPOSITION, PRODUCTION METHOD THEREOF AND USE OF SAME

(30) Priorité: 18.08.2005 FR 0508602
(43) Date de publication de la demande: 11.06.2008
(73) Titulaire: Spécialités pour Industries Alimentaires en Abrege Spindal, 77220 Gretz Armainvilliers (FR)
(72) Inventeur: VIVAS, Nicolas, FR-77220 Gretz Armainvilliers (FR); NEDJMA, Mustapha, FR-77220 Gretz Armainvilliers (FR); ZANARDELLI, Pietro, FR-77220 Gretz Armainvilliers (FR)
(74) Mandataire: Fosse, Danièle
(86) Numéro de dépôt international: PCT/FR2006/001929
(87) Numéro de publication internationale: WO 2007/020348

(56) Documents cités:
- SANTOS-BUELGA C ET AL: "PROANTHOCYANIDINS AND TANNIN-LIKE COMPOUNDS - NATURE, OCCURRENCE, DIETARY INTAKE AND EFFECTS ON NUTRITION AND HEALTH" JOURNAL OF THE SCIENCE OF FOOD AND AGRICULTURE, WILEY & SONS, CHICHESTER, GB, vol. 80, no. 7, 15 mai 2000 (2000-05-15), pages 1094-1117, XP000927238 ISSN: 0022-5142
- TIMBERLAKE C F ET AL: "INTERACTIONS BETWEEN ANTHO CYANINS PHENOLIC COMPOUNDS AND ACETALDEHYDE AND THEIR SIGNIFICANCE IN RED WINES" AMERICAN JOURNAL OF ENOLOGY AND VITICULTURE, vol. 27, no. 3, 1976, pages 97-105, XP009066195 ISSN: 0002-9254
- ATANASOVA VESSELA ET AL: "Effect of oxygenation on polyphenol changes occurring in the course of wine-making" ANALYTICA CHIMICA ACTA, vol. 458, no. 1, 29 avril 2002 (2002-04-29), pages 15-27, XP002380076 ISSN: 0003-2670
- ES-SAFI NOUR-EDDINE ET AL: "Flavanols and anthocyanins as potent compounds in the formation of new pigments during storage and aging of red wine" ACS SYMPOSIUM SERIES AMER CHEMICAL SOC, 1155 SIXTEENTH ST NW, WASHINGTON, DC 20036 USA SERIES : ACS SYMPOSIUM SERIES (ISSN 0097-6156(PRINT)), 2004, pages 143-159, XP009066196 & 225TH NATIONAL MEETING OF THE AMERICAN-CHEMICAL-SOCIETY; NEW ORLEANS, LA, USA; MARCH 23 -27, 2003 ISSN: 0-8412-3900-2(H)
- VIDAL S ET AL: "Taste and mouth-feel properties of different types of tannin-like polyphenolic compounds and anthocyanins in wine" ANALYTICA CHIMICA ACTA 18 JUN 2004 NETHERLANDS, vol. 513, no. 1, 18 juin 2004 (2004-06-18), pages 57-65, XP002380077 ISSN: 0003-2670
- FINE A M: "OLIGOMERIC PROANTHOCYANIDIN COMPLEXES: HISTORY, STRUCTURE, AND PHYTOPHARMACEUTICAL APPLICATIONS" ALTERNATIVE MEDICINE REVIEW, THORNE RESEARCH INC., SANDPOINT,, US, vol. 5, no. 2, 2000, pages 144-151, XP009012020 ISSN: 1089-5159

## Description

La présente invention a trait à une composition de tanins, à son procédé de préparation ainsi qu'à ses diverses applications.

La présente invention a plus spécialement trait à une composition nouvelle de tanins proanthocyanidiques extraits notamment de pépins de raisins, à son procédé de préparation par la maîtrise des paramètres de réactions d'additions nucléophiles entre un alkanal à courte chaîne et lesdits proanthocyanidols ainsi qu'à ses nombreuses applications industrielles, en particulier en agroalimentaire, en oenologie, ainsi que dans les domaines cosmétique et/ou pharmaceutique.

### ARRIERE-PLAN TECHNIQUE

On connaît, depuis les travaux de Laborde dans les années 1905 - 1910, le rôle joué par l'éthanal dans la polymérisation des tanins. Le principe de la réaction est assez simple, puisqu'un aldéhyde en milieu acide est utilisé comme agent de polymérisation de substances de nature phénolique. On connaît les matières synthétiques produites à partir de phénols et de méthanal.

Cependant, la réaction, si elle est réalisée en présence d'un excès d'aldéhyde, conduit à la formation d'un polymère insoluble. En oenologie, des travaux très abondants ont eu pour objet d'étudier ce type de réaction.

Dans le vin, les réactions oxydatives sont générales et présentes à tout moment de la transformation du vin à son traitement et à son conditionnement.

L'élevage constitue traditionnellement le moment le plus propice aux oxydations servant à la stabilisation de la matière colorante et à la polymérisation des tanins. On sait en effet que, dans les vins rouges en particulier, la matière colorante sous forme libre est peu stable et sensible aux variations de pH, à l'oxygène et au SO₂.

Les associations de la matière colorante avec les tanins permettent à la fois de stabiliser la couleur et de la rendre moins sensible aux conditions du milieu.

De la même manière, l'augmentation du degré de polymérisation des tanins lors de phases oxydatives permet de diminuer leur astringence et donc d'améliorer les qualités gustatives des vins.

La réaction est relativement classique Il s'agit d'une autoxydation couplée débutant par la consommation de l'oxygène dissous par les composés phénoliques, suivie de la formation d'hydroperoxyde d'hydrogène agissant en oxydant l'éthanol en éthanal. L'éthanal ainsi formé au pH acide du vin agit comme nucléophile et permet la polymérisation, soit des tanins entre eux, soit des tanins avec les anthocyanes. Le fait le plus remarquable est l'assombrissement de la couleur rouge du vin et son évolution vers les nuances bleutées caractéristiques de ce type de réaction.

### PROBLEME(S) POSE(S)

Le type de réaction décrite ci-dessus est fortement consommatrice de temps et nécessite le recours à l'oxygène.

La sensibilité à l'oxygène des vins ainsi que les conséquences négatives d'un élevage trop long sont bien connues. Dans les vins rouges le rapport tanins sur anthocyanes est un facteur déterminant de son aptitude à subir un élevage oxydatif. Pour certains d'entre eux, ce rapport très élevé (> 5) ne permet pas d'espérer par le mécanisme classique d'élevage une stabilisation de la matière colorante. Il est alors utile de conserver les vins avec le moins d'oxygène et pour des durées courtes. D'autre part, il existe des vins naturellement pauvres en tanins qui ne peuvent pas supporter d'étapes oxydatives trop importantes. Dans ce cas-là, toutes mauvaises estimations du management de l'élevage se traduisent par une précipitation des tanins, une évolution prématurée de la couleur et l'apparition en bouche de maigreur et de sécheresse ; dans le même temps, l'arôme de fruit s'estompe et disparaît même dans les cas les plus extrêmes.

Plus généralement les recherches dans ce domaine visent à résoudre un certain nombre de problèmes et/ou à améliorer des solutions techniques existantes, en particulier :
- la stabilisation et/ou l'intensification de la couleur des vins
- l'amélioration des qualités gustatives des vins
- le conditionnement des compositions de tanins proanthocyanidiques
- la solubilisation de ces compositions dans les vins.

Plusieurs solutions se présentent pour répondre aux effets présentés ci-dessus : par l'ajout de macromolécules telles que des polysaccharides levuriens ou de la gomme arabique. On augmente l'impression de rondeur des vins en enrobant les tanins par les chaînes de polysaccharides. Mais ce phénomène bien qu'intéressant ne participe pas à la stabilisation des tanins du vin et de sa couleur. En fait, il s'agit plus d'une opération de maquillage à pratiquer sur le produit fini. Il existe des méthodes de tanisage permettant de corriger les déséquilibres structurels du vin et les rendre plues aptes à l'élevage oxydatif. Cependant, cette seconde voie n'est pas systématiquement applicable en particulier dans les vins fragiles de part leurs origines et leurs destinations. C'est le cas des vins rosés et des vins rouges très peu tanniques, fortement colorés et avec un arôme de fruit puissant qui en fait son principal attrait. Pour ces vins, toute oxydation conduit à une perte d'une partie de leur qualité.

### SOLUTION TECHNIQUE DE L'INVENTION

Dans le but de résoudre les problèmes techniques évoqués ci-dessus, la présente invention procure une composition de tanins proanthocyanidiques qui se caractérise en ce qu'elle comporte au moins 25 % et jusqu'à 60 % (P/P) de proanthocyanidols oligomères reliés entre eux par des ponts alkyles en C₂-C₅ : dans la présente invention ces derniers sont appelés « tanalkyl », le « tanéthyl » représentant le cas où ces derniers sont reliés entre eux par un pont éthyle.

Dans le cadre de la présente invention on entend par oligomères, un polymère constitué de 2 à 10 unités répétées, de manière aléatoire, d'un ou de plusieurs monomères soit catéchol, épicatéchol et épicatéchol gallate pour les procyanidols, soit gallocatéchol, épigallocatéchol, épigallocatéchol gallate pour les prodelphinidols ; tous des flavan-3-ols.

La composition de tanins selon l'invention répond en tout point aux normes anciennes et nouvelles sur les tanins oenologiques établies par l'OIV dans son codex. En particulier, il s'agit :
- de sa teneur en polyphénols totaux supérieurs à 65 %,
- de sa capacité à donner une réponse positive à la réaction de Bate-Smith (butanolyse chlorhydrique en présence de sels de fer) caractérisant le produit comme un proanthocyanidol,
- de sa teneur en matière active (encore appelée tanins vrais),
- de son respect des limites en métaux,
- de sa capacité à floculer les protéines, et
- de sa couleur chamois qui répond aux limites liées à la couleur des tanins.

Utilisée en tant qu'adjuvant ou co-adjuvant oenologique en particulier dans des vins rosés ou dans des vins rouges jeunes, la composition selon l'invention donne des résultats remarquables en termes notamment de stabilisation rapide de la couleur en provoquant un accroissement de près de 50 % des associations tanins-anthocyanes.

En dessous de 25 % en proanthocyanidols oligomères reliés entre eux par alkyl en C₂-C₅, la composition présente les inconvénients de nécessiter de fortes doses d'emploi pour présenter l'efficacité du produit. Dans ce cas il s'agit d'avantage d'un tannisage classique que d'un traitement au tanalkyl.

Au-delà de 60 %, se pose le problème de risque d'insolubilisation d'une partie du produit par surpolymérisation. Sur un produit trop polymérisé , les réactions attendues deviennent plus faibles et plus lentes. En effet, les formes de haut poids moléculaires sont moins réactives et donc agissent avec moins d'intensité que leurs homologues de degré de polymérisation plus faible.

Le mode d'action de la composition selon l'invention est expliqué ci-après.

L'ajout, dans un milieu contenant des anthocyanes, de la composition conforme à l'invention conduit rapidement et de manière spontanée à la formation d'associations tanins-anthocyanes.

La composition selon l'invention a l'aptitude de se dépolymériser pour reformer ensuite avec d'autres polyphénols, principalement les tanins eux-mêmes et les anthocyanes, de nouvelles associations.

La composition de tanins selon l'invention présente les caractéristiques complémentaires suivantes :
* les proanthocyanidols oligomères sont constitués d'unités de flavane-3-ol (catéchol et/ou epicatéchol)).
   II est possible de produire à partir de procyanidols un tanalkyl (c'est-à-dire des empilements de catéchol, d'épicatéchol et d'épicatéchol gallate) mais également à partir de prodelphinidols (c'est-à-dire des empilements de gallocatéchol, d'épigallocatéchol et d'épigallocatéchol gallate). Ces matières premières peuvent être issues des pépins, des pellicules et de la rafle des raisins, des sarments de vigne, d'écorces de chêne, châtaignier, de pin maritime ; sont exclus les tanins présentant des structures de type résorcinol comme les profisétinidols, prorobinétinidols issus de bois exotiques en particulier le quebracho ou du bois d'acacia ou de mimosa.
* elle comporte au moins 25 % et jusqu'à 60 % de proanthocyanidols oligomères reliés entre eux par des ponts éthyles

La présente invention a encore pour objet un procédé de préparation de ladite composition de tanins qui se caractérise en ce qu'il comporte une étape consistant à faire réagir en présence d'éthanol des proanthocyanidols oligomères et un alkanal en C₂-C₅.

D'autres caractéristiques complémentaires ou alternatives du procédé de préparation de la composition conforme à l'invention sont les suivantes :
* la concentration d'éthanol est de 7% à 13 % (V/V), de préférence 10 % (V/V).
   Une concentration d'éthanol inférieure à 7 % (V/V) n'assure pas une solubilisation des tanins, surtout lorsqu'ils sont fortement concentrés (200 g/l à 300 g/l), ni des adduits alkyl peu solubles dans l'eau. Au-delà de 13 %, les surcoûts liés au volume d'éthanol à utiliser et à régénérer ne se justifient par aucune amélioration technique.
* les concentrations respectives d'alkanal en C₂-C₅ et de proanthocyanidols oligomères varient de 1 à 2

Dans le cas de la mise en oeuvre de solutions de proanthocyanidols oligomères de 15 g/l à 30 g/l, on peut utiliser une concentration d'alkanal en C₂-C₅ deux fois supérieure. Avec des milieux dix fois plus concentrés en proanthocyanidols oligomères, il est avantageux de limiter le rapport des concentrations alkanal en C₂-C₅/proanthocyanidols de 1 à 1,5.
* le pH de la réaction est ajusté à une valeur de 2 à 4, de préférence 3.

Une valeur de pH inférieure à 2 accélère la réaction mais il y a des risques de précipitations (production de polymères insolubles) donc de perte de rendement du procédé.

Une valeur supérieure à 4 entraîne une oxydabilité accrue du milieu et la destruction d'une partie de la matière active.

L'ajustement du pH autour de la valeur 3, préférée, peut être réalisé avec un acide ou un mélange d'acides organiques dont l'acide tartrique, par exemple. Toutefois, leur utilisation présente l'inconvénient majeur de leur élimination ultérieure dans les étapes subséquentes du procédé de préparation et de conditionnement de la composition.

Aussi, l'utilisation pour ajuster le pH d'acides minéraux forts, tels que HCl 12N par exemple, est particulièrement préférée car elle permet de diminuer les volumes d'acide mis en oeuvre pour atteindre les pH souhaités.
* la température de la réaction est maintenue à une valeur de 10°C à 20°C.
   En dessous de 7°C, la réaction est pratiquement stoppée, au-delà de 20°C, un emballement de la réaction est noté et aucune amélioration de la rentabilité de la réaction n'est observée.

Les proanthocyanidols oligomères utiles dans le cadre de la présente invention peuvent être obtenus à partir d'un extrait végétal, tel que par exemple écorce de châtaignier, chêne, pin maritime, pépins, rafle ou pellicules de raisins, sarments de vigne.

Dans le cadre de la présente invention, il est particulièrement avantageux d'utiliser des proanthocyanidols oligomères extraits de pépins de raisins.
* Selon un mode avantageux de réalisation du procédé selon l'invention, l'alkanal en C₂-C₅ est l'éthanal
   * le procédé selon l'invention comporte en outre les étapes consistant à concentrer puis à sécher la composition obtenue
* l'étape de concentration peut être réalisée par centrifugation, filtration, ultrafiltration, évaporation, de préférence évaporation ; la température est avantageusement contrôlée autour de 40°C
* l'étape d'évaporation est réalisée sous vide à une température inférieure ou égale à 40°C
* l'étape de séchage de la composition de l'invention est réalisée par atomisation ou lyophilisation , de préférence atomisation; avantageusement, l'étape de séchage est réalisée pratiquement immédiatement après l'étape de concentration
* l'étape d'atomisation de la composition de l'invention est réalisée de manière avantageuse en présence d'une quantité efficace d'un agent permettant sa granulation et favorisant sa solubilisation ultérieure ; de préférence un tel agent est la maltodextrine ou la gomme arabique.

La présente invention a encore pour objet une utilisation de ladite composition de tanins dans les domaines techniques suivants :
- agroalimentaire, en particulier dans la stabilisation de la couleur dans tout aliment contenant des anthocyanes, notamment les colorants rouges dans les produits laitiers et dérivés, dans le vinaigre ou dans des boissons non alcoolisées tel que jus de raisins
- extraction des anthocyanes pour stabiliser les anthocyanes libres du mélange au fur et à mesure de l'extraction et augmenter le pouvoir colorant par formation de combinaison tanins-éthyl-anthocyanes
- oenologie, comme adjuvant ou co-adjuvant -voir exemples ci-après- et produits issus de la distillation (modification de leur couleur, diminution du brûlant de l'alcool) et
- cosmétique et/ou pharmaceutique, en particulier pour leur propriété antioxydante, et plus précisément comme actif piégeant les radicaux libres.

La présente invention a enfin pour objet l'utilisation de ladite composition de tanins dans la préparation d'un médicament destiné à un traitement thérapeutique, en particulier, pour améliorer la circulation sanguine.

La présente invention va maintenant être décrite de manière plus détaillée dans ses objets et avantages et à l'aide d'exemples de réalisation donnés à titre purement illustratif et non limitatif ainsi que des dessins annexés dans lesquels :
- la figure 1 illustre des réactions d'additions nucléophiles entre tanins et anthocyanes par l'éthanal,
- la figure 2 montre la formation de combinaisons tanins-anthocyanes à partir de proanthocyanidol-éthyl-proanthocyanidol,
- la figure 3 schématise le mode d'action de la composition selon l'invention,
- la figure 4 illustre un exemple de spectre de masse en électronébulisation montrant la présence d'une unité élémentaire de l'oligomère de la composition conforme à l'invention et
- la figure 5 illustre un exemple de spectre de résonance magnétique nucléaire d'une unité élémentaire de l'oligomère de la composition conforme à l'invention.

### DESCRIPTION DETAILLEE DE L'INVENTION

1. Procédé de préparation d'une composition conforme à l'invention à partir de proanthocyanidols de pépins de raisins et d'éthanal
   1.1 Procédé de fabrication de l'extrait de pépins de raisins pour obtention des proanthocyanidols
      Les proanthocyanidols sont obtenus après broyage de pépins issus de raisins blancs à maturité et extraits par une solution hydroalcoolique (environ 20% d'éthanol) en pH acide (pH<4) et conditionnés après lyophilisation ou atomisation. Le produit obtenu est riche en procyanidols dont la masse varie de 600 à 4500 Da.
   1.2 Conditions opératoires de l'étape de réaction entre proanthocyanidols et éthanal en présence d'éthanol
      1.2.1 On fait réagir des solutions de proanthocyanidols de pépins de raisins telle que préparée au point 1.1 : 20 g/l avec 40 g/l d'éthanal en présence de 10 % (V/V) d'éthanol
      1.2.2 On fait réagir des solutions de proanthocyanidols de pépins de raisins telle que préparée au point 1.1 : 250 g/l avec 312,5 g/l d'éthanal en présence de 10 % (V/V) d'éthanol
      1.2.3 La température de la réaction est de 15°C

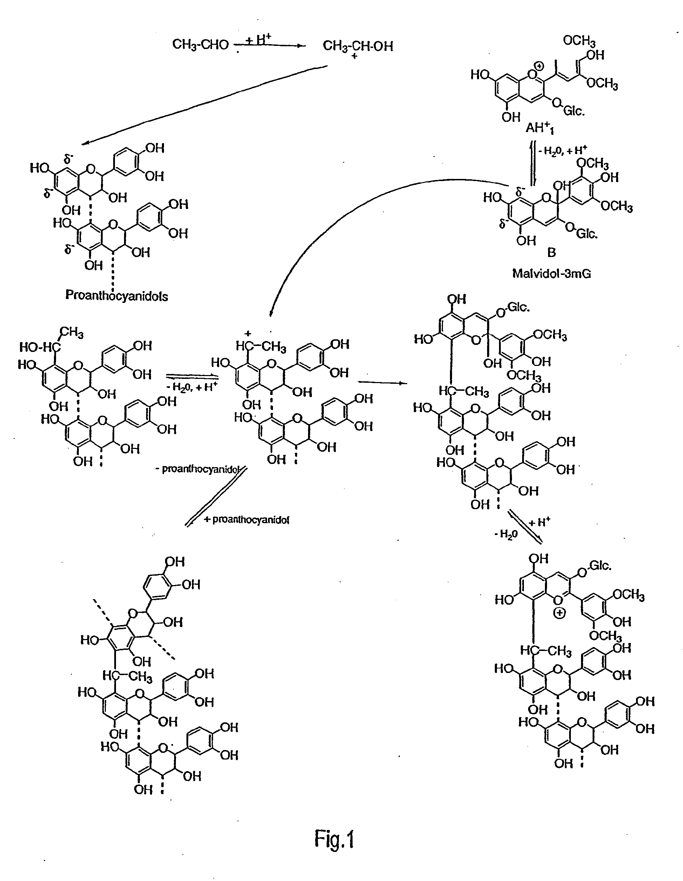

      La figure 1 illustre des réactions d'additions nucléophiles entre tanins et anthocyanes par l'éthanal.

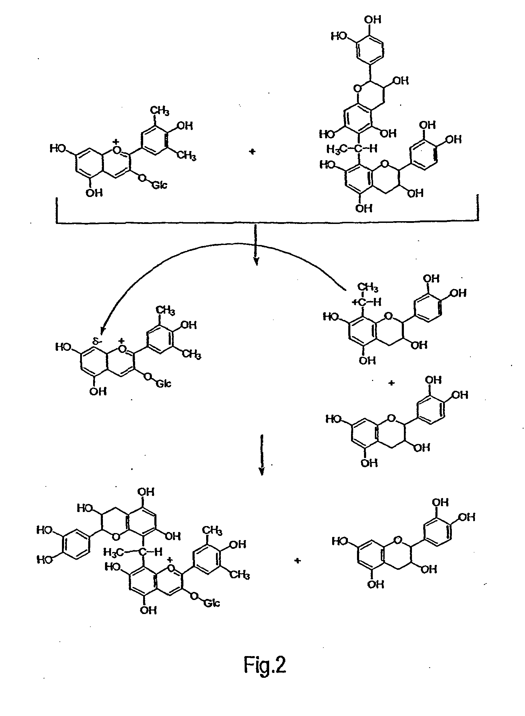

      La figure 2 montre la formation des associations tanins-anthocyanes à partir d'une composition conforme à la présente invention comportant le produit de la réaction proanthocyanidol-éthyl-proanthocyanidol décrite au point 1.2.1
   1.3 Etapes subséquentes de concentration et d'atomisation
   1.4 Caractérisation quantitative et qualitative de la composition conforme à l'invention
      1.4.1 Présence dans la composition selon l'invention dans des proportions variables de 25 % à 60 % d'associations entre proanthocyanidols oligomères de tanins de pépins de raisins par l'intermédiaire de ponts éthyles :
      1.4.2 Contrôle de la composition par électronébulisation et spectrométrie de masse

Le contrôle de la composition conforme à la présente invention peut être réalisé simplement par spectrométrie de masse en utilisant la technique dite de l'électronébulisation (electrospray), soit la LSIMS (liquid secondary ion mass spectrometry) en utilisant des voltages volontairement élevés pour favoriser la fragmentation des proanthocyanidols oligomères reliés par des ponts éthyles de la composition de l'invention et l'identification de son unité structurale élémentaire composée de flavane-3-ol sur lequel est attaché le radical éthyle et un autre flavane-3-ol. Cette unité répond à m/z de 607 [M-H]⁻.

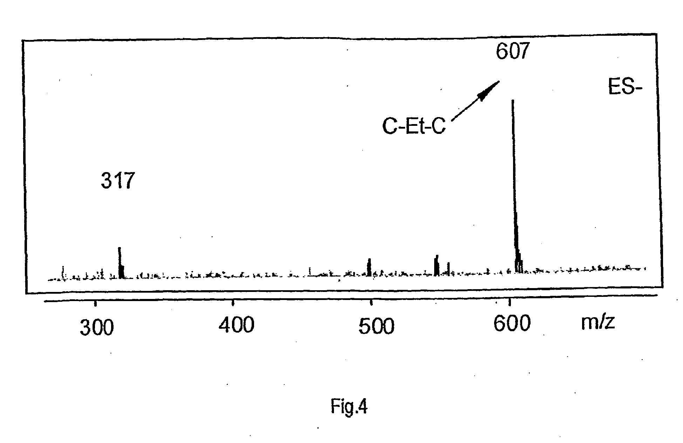

Ainsi, la figure 4 montre un exemple de spectre de masse, mode négatif (ES-), d'un contrôle de présence de la composition selon l'invention dans un mélange réactionnel. Le pic à 607 correspond aux unités élémentaires de flavane-3-ol reliées entre elles par un pont éthyle. Ce spectre a été obtenu par un ES à haut cône de voltage pour casser les oligomères.

Il est également possible d'estimer la qualité de la composition selon l'invention par une résonance magnétique du proton (RMN) nécessitant dans le cas d'un appareil fonctionnant respectivement à 250 MHz ou à 400 MHz, 30 min ou 10 min. Dans ce cas on repère les signaux caractéristiques du C-H de la liaison Carbone-Carbone entre les tanins et l'éthanal central ainsi que le signal du CH₃ libre du radical éthyle.

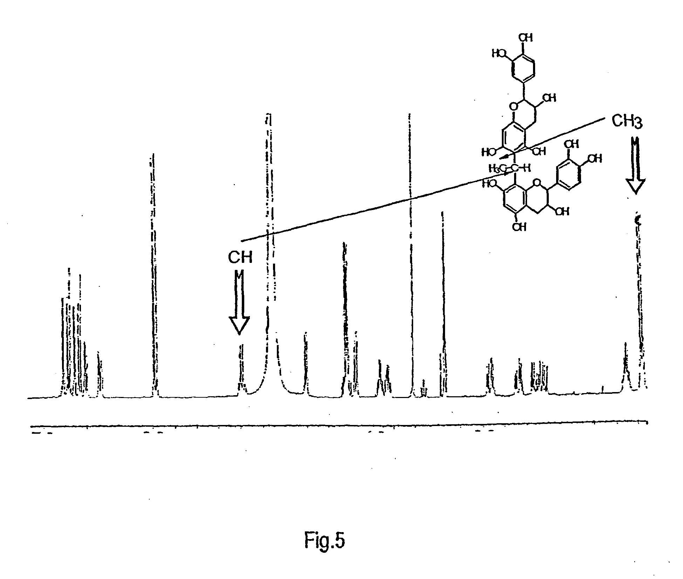

Comme illustré à la figure 5 - avec un appareil Bruker DPX, 400 MHz)- ces signaux apparaissent respectivement à 5,2 ppm et 1,55 ppm.

Par ailleurs, avec la RMN et par intégration de l'aire des signaux, il est possible d'estimer le poids de matière active dans la composition selon l'invention.

### 2. Application de la composition conforme à l'invention

### 2.1 Oenologie

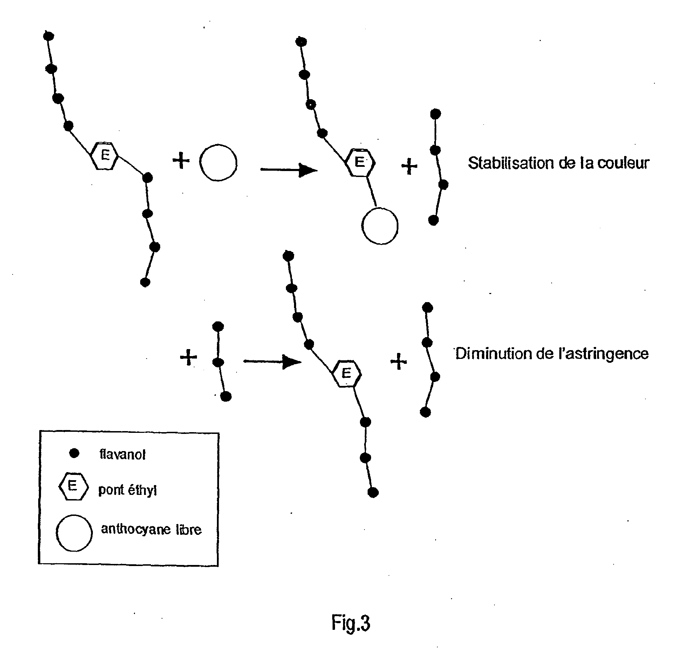

La figure 3 illustre un mode d'action de la composition conforme à l'invention, utilisée comme actif oenologique.

A titre d'illustration, les inventeurs ont supplémenté un vin rouge de Merlot noir de l'année 2003 (Tableau 1) et un vin rouge de Merlot noir de 5 ans (Tableau 2) en composition conforme à la présente invention et ce, pour différentes concentrations de ladite composition (250 mg/l , 500 mg/l et 1000 mg/l).

Après 7 jours de temps de réaction, les analyses phénoliques classiques et chromatiques sur ces deux vins ont été effectuées et comparées à un témoin (T), c'est-à-dire à un vin n'ayant subi aucun traitement ni ajout.

**TABLEAU 1**

| Merlot noir 2003 Après 7 j de réaction | | T | +250 mg/l | +500 mgll | +1000 mg/l |
|---|---|---|---|---|---|
| Composés Phénoliques | d₂₀₀ | 59 | 63 | 65 | 70 |
| | % combinaisons Tanins-Anthocyanes | 27 | 41 | 44 | 46 |
| | Tanins g/l | 2,96 | 3,10 | 3,17 | 3,46 |
| Couleur | Intensité colorante | 1,23 | 1,28 | 1,32 | 1,42 |
| | Teinte | 0,56 | 0,59 | 0,62 | 0,65 |
| | d_{420%} | 32,9 | 33,3 | 33,1 | 33,1 |
| | d_{529%} | 56,2 | 54,5 | 54,0 | 52,5 |
| | d_{620%} | 10,9 | 12,2 | 12,9 | 14,7 |
| | DA% | 62,0 | 59,3 | 58,4 | 55,0 |

**TABLEAU 2**

| Merlot noir 1999 Après 7 j de réaction | | T | +250 mg/l | +500 mg/l | +1000 mg/l |
|---|---|---|---|---|---|
| Composés Phénoliques | d₂₈₀ | 48 | 51,8 | 53,9 | 58,1 |
| | % combinaisons Tanins-Anthocyanes | 89 | 90 | 91 | 95 |
| | Tanins g/l | 3,17 | 3,44 | 3,54 | 3,78 |
| Couleur | Intensité colorante | 1,20 | 1,31 | 1,33 | 1,37 |
| | Teinte | 0,66 | 0,77 | 0,79 | 0,81 |
| | d_{420%} | 35,6 | 39,6 | 39,9 | 40,0 |
| | d_{520%} | 52,0 | 48,9 | 48,7 | 48,4 |
| | d_{620%} | 12,4 | 11,5 | 11,4 | 11,6 |
| | DA% | 55,0 | 48,1 | 48,0 | 47,9 |

- Sur le vin vieux (5 ans), les résultats montrent clairement que la composition de l'invention n'a qu'un faible effet sur ce vin dont la majorité de la couleur est déjà stabilisée. Cependant on peut noter tout de même des augmentations appréciables de l'intensité colorante, malheureusement suivies d'une baisse de la nuance bleue sombre de la couleur (d620%), de l'impression rouge (dA%) et une augmentation du caractère tuilé du vin (teinte).
- En revanche, lorsque le vin nécessite une stabilisation de sa matière colorante, comme c'est le cas du vin jeune, on observe d'abord et en seulement une semaine :
   - l'augmentation de 10% à 50% de pourcentage d'associations Tanins-Anthocyanes,
   - une augmentation de l'intensité colorante,
   - une augmentation appréciable de la couleur bleue sombre du vin
   - avec une variation très limitée de la teinte.

En réalité le prolongement de l'expérience sur une semaine supplémentaire montre que les résultats s'accentuent dans le temps pour se stabiliser au bout d'une vingtaine de jours.

Dans les vins rosés, le résultat est encore plus spectaculaire puisque le pourcentage de combinaisons Tanins-Anthocyanes inférieur à 15 % passe à près de 50 % ; avec une stabilisation de la couleur, qui devient particulièrement résistante aux variations de pH, à l'ajout de SO₂ et aux effets destructeurs de la chaleur et de la lumière.

Ces résultats sont remarquables comparés à ceux obtenus avec des adjuvants ou co-adjuvants classiques qui nécessitent le recours aux phases oxydatives et une durée d'action de plusieurs mois.

## Revendications

1. Composition de tanins proanthocyanidiques comportant au moins 25 % et jusqu'à 60 % (P/P) de proanthocyanidols oligomères reliés entre eux par des ponts alkyles en C₂-C₅.

2. Composition de tanins selon la revendication 1, **caractérisée en ce que** les proanthocyanidols oligomères sont constitués de 2 à 10 unités de flavane-3-ol.

3. Procédé de préparation d'une composition de tanins selon la revendication 1 ou 2, **caractérisé en ce qu'**il comprend une étape consistant à faire réagir en présence d'éthanol des proanthocyanidols oligomères et un alkanal en C₂-C₅.

4. Procédé selon la revendication 3, **caractérisé en ce que** la concentration d'éthanol est de 7% à 13 % (V/V).

5. Procédé selon la revendication 3 ou 4, **caractérisé en ce que** les concentrations respectives d'alkanal en C₂-C₅ et de proanthocyanidols oligomères varient de 1 à 2.

6. Procédé selon l'une quelconque des revendications 3 à 5, **caractérisé en ce que** le pH de la réaction est ajusté à une valeur de 2 à 4.

7. Procédé selon l'une quelconque des revendications 3 à 6, **caractérisé en ce que** la température de la réaction est maintenue à une valeur de 10°C à 20°C.

8. Procédé selon l'une quelconque des revendications 3 à 7, **caractérisé en ce que** les proanthocyanidols oligomères sont produits à partir d'un extrait végétal choisi dans le groupe constitué par l'écorce de châtaignier, de chêne, de pin maritime, ou à partir de pépins ou de pellicules de raisins.

9. Procédé selon la revendication 8, **caractérisé en ce que** les proanthocyanidols oligomères sont produits à partir de pépins de raisins.

10. Procédé selon l'une quelconque des revendications 3 à 9, **caractérisé en ce que** ledit alkanal est l'éthanal.

11. Procédé selon l'une quelconque des revendications 3 à 10, **caractérisé en ce qu'**il comporte en outre les étapes consistant à concentrer puis à sécher la composition obtenue.

12. Procédé selon la revendication 11, **caractérisé en ce que** l'étape de concentration est réalisée par évaporation sous vide à une température inférieure ou égale à 40°C.

13. Procédé selon la revendication 11 ou 12, **caractérisé en ce que** l'étape de séchage est réalisée par atomisation.

14. Composition selon la revendication 1 ou 2 ou obtenue par un procédé selon l'une quelconque des revendications 3 à 13, **caractérisé en ce qu'**elle comporte au moins 25 % et jusqu'à 60 % de proanthocyanidols oligomères reliés entre eux par des ponts éthyles.

15. Utilisation d'une composition selon l'une quelconque des revendications 1, 2, ou 14 ou obtenue par un procédé selon l'une quelconque des revendications 3 à 13, comme adjuvant ou co-adjuvant oenologique et/ou pour des produits issus de la distillation.

16. Utilisation d'une composition selon l'une quelconque des revendications 1, 2, ou 14 ou obtenue par un procédé selon l'une quelconque des revendications 3 à 13, comme agent stabilisant la couleur en agroalimentaire.

17. Utilisation d'une composition selon l'une quelconque des revendications 1, 2, ou 14 ou obtenue par un procédé selon l'une quelconque des revendications 3 à 13, comme antioxydant en cosmétique.

18. Utilisation d'une composition selon l'une quelconque des revendications 1, 2, ou 14 ou obtenue par un procédé selon l'une quelconque des revendications 3 à 13, dans la préparation d'un médicament destiné à un traitement thérapeutique notamment en vue d'améliorer la circulation sanguine.

## Claims

1. Proanthocyanidin tannin composition comprising at least 25% and up to 60% (w/w) oligomeric proanthocyanidols interconnected by alkyl bridges at C₂-C₅.

2. Tannin composition of claim 1, **characterised in that** the oligomeric proanthocyanidols consist of 2 to 10 flavan-3-ol units.

3. Method of preparing a tannin composition as claimed in claim 1 or 2, **characterised in that** it includes a step consisting in reacting oligomeric proanthocyanidols and an alkanal at C₂-C₅ in the presence of ethanol.

4. Method of claim 3, **characterised in that** the ethanol concentration is from 7% to 13% (V/V).

5. Method as claimed in claim 3 or 4, **characterised in that** the respective concentrations of alkanal at C₂-C₅ and of oligomeric proanthocyanidols vary from 1 to 2.

6. Method as claimed in any of claims 3 to 5, **characterised in that** the pH of the reaction is adjusted to a value of 2 to 4.

7. Method as claimed in any of claims 3 to 6, **characterised in that** the reaction temperature is maintained at a value of 10°C to 20°C.

8. Method as claimed in any of claims 3 to 7, **characterised in that** the oligomeric proanthocyanidols are produced from a vegetable extract chosen from the group consisting of chestnut, oak or maritime pine bark or from grape seeds or skin.

9. Method of claim 8, **characterised in that** the oligomeric proanthocyanidols are produced from grape seeds.

10. Method of claimed in any of claims 3 to 9, **characterised in that** said alkanal is ethanal.

11. Method as claimed in any of claims 3 to 10, **characterised in that** it further comprises the steps consisting in concentrating and then drying the resulting composition.

12. Method of claim 11, **characterised in that** the concentrating step is carried out by vacuum evaporation at a temperature less than or equal to 40°C.

13. Method as claimed in claim 11 or 12, **characterised in that** the drying step is carried out by

14. Composition as claimed in claim 1 or 2 or obtained by a method as claimed in any of claims 3 to 12, **characterised in that** it comprises at least 25% and up to 60% oligomeric proanthocyanidols interconnected by ethyl bridges.

15. Use of a composition as claimed in any of claims 1, 2 or 14 or obtained by a method as claimed in any of claims 3 to 13, as an oenological adjuvant or co-adjuvant and/or for products derived from distillation.

16. Use of a composition as claimed in any of claims 1, 2 or 14 or obtained by a method as claimed in any of claims 3 to 13, as a colour stabiliser in the agri-food industry.

17. Use of a composition as claimed in any of claims 1, 2 or 14 or obtained by a method as claimed in any of claims 3 to 13, as an antioxidant in the cosmetics industry.

18. Use of a composition as claimed in any of claims 1, 2 or 14 or obtained by a method as claimed in any of claims 3 to 13, in the preparation of a drug intended for a therapeutic treatment, in particular with a view to improving blood circulation.

## Patentansprüche

1. Proanthocyanidin-Tanninzusammensetzung, die mindestens 25 Gew.-% und bis zu 60 Gew.-% oligomere Proanthocyanidole umfasst, die durch C₂-C₅-Alkylbrücken miteinander verbunden sind.

2. Tanninzusammensetzung nach Anspruch 1, **dadurch gekennzeichnet, dass** die oligomeren Proanthocyanidole aus 2 bis 10 Flavan-3-ol-Einheiten bestehen.

3. Verfahren zur Herstellung einer Tanninzusammensetzung nach Anspruch 1 oder 2, **dadurch gekennzeichnet, dass** es einen Schritt umfasst, der im Reagierenlassen der oligomeren Proanthocyanidole und eines C₂-C₅-Alkanals in Gegenwart von Ethanol besteht.

4. Verfahren nach Anspruch 3, **dadurch gekennzeichnet, dass** die Konzentration des Ethanols 7 Vol.-% bis 13 Vol.-% beträgt.

5. Verfahren nach Anspruch 3 der 4, **dadurch gekennzeichnet, dass** die jeweiligen Konzentrationen des C₂-C₅-Alkanals und der oligomeren Proanthocyanidole von 1 bis 2 variieren.

6. Verfahren nach einem der Ansprüche 3 bis 5, **dadurch gekennzeichnet, dass** der pH-Wert des Reaktionsgemischs auf einen Wert von 2 bis 4 eingestellt wird.

7. Verfahren nach einem der Ansprüche 3 bis 6, **dadurch gekennzeichnet, dass** die Temperatur des Reaktiorisgemischs auf einem Wert von 10 °C bis 20 °C gehalten wird.

8. Verfahren nach einem der Ansprüche 3 bis 7, **dadurch gekennzeichnet, dass** die oligomeren Proanthocyanidole aus einem pflanzlichen der aus der Gruppe bestehend aus Kastanienrinde, Eichenrinde, Strandkieferrinde ausgewählt ist, oder aus Traubenkernen oder -häuten hergestellt sind.

9. Verfahren nach Anspruch 8, **dadurch gekennzeichnet, dass** die oligomeren Proanthocyanidole aus Traubenkernen hergestellt sind.

10. Verfahren nach einem der Ansprüche 3 bis 9, **dadurch gekennzeichnet, dass** das genannte Alkanal ein Ethanal ist.

11. Verfahren nach einem der Ansprüche 3 bis 10, **dadurch gekennzeichnet, dass** es außerdem Schritts umfasst, die im Konzentrieren und dann Trocknen der gewonnenen Zusammensetzung bestehen.

12. Verfahren nach Anspruch 11, **dadurch gekennzeichnet, dass** der Konzentrationsschritt mittels Vakuumverdampfung bei einer Temperatur, die niedriger als oder gleich 40 °C ist, durchgeführt wird.

13. Verfahren nach Anspruch 11 oder 12, **dadurch gekennzeichnet, dass** der Trocknungsschritt mittels Zerstäubung durchgeführt wind.

14. Zusammensetzung nach Anspruch 1 oder 2 oder durch ein Verfahren nach einem der ansprüche 3 bis 13 gewonnene Zusammensetzung, **dadurch gekenntzeichnet, dass** sie mindestens 25 Gew.-% und bis zu 60 Ges.-% oligomere Proanthocyanidole umfasst, die durch Ethylbrücken miteinander verbinden sind.

15. Verwendung einer Zusammensetzung nach einem der Ansprüche 1, 2 oder 14 oder durch ein Verfahren nach einem der Ansprüche 3 bis 13 gewonnene Zusammensetzung als önologisches Adjuvans oder Koadjuvans und/oder für Produkte, die aus einer Destillation hervorgehen.

16. Verwendung einer Zusammensetzung nach einem der Ansprüche 1, 2 oder 14 oder durch ein Verfahren nach einem der Ansprüche 3 bis 13 gewonnene Zusammensetzung als Lebensmittelfarbstoff-Stabilisator.

17. Verwendung einer Zusammensetzung nach einem der Ansprüche 1, 2 oder 14 oder durch ein Verfahren nach einem der Ansprüche 3 bis 13 gewonnene Zusammensetzung als Antioxidans in der Kosmetik.

18. Verwendung einer Zusammensetzung nach einem der Ansprüche 1, 2 oder 14 oder durch ein Verfahren nach einem der Ansprüche 3 bis 13 gewonnene Zusammensetzung zur Herstellung eines Arzneimittels, das zur therapeutischen Behandlung bestimmt ist, insbesondere zur Verbesserung des Blutkreislaufs.
